Europäisches Patentamt

European Patent Office

Office européen des brevets

(11) Publication number: **0 378 383 A2**

(12) **EUROPEAN PATENT APPLICATION**

(21) Application number: **90300271.5**

(22) Date of filing: **10.01.90**

(51) Int. Cl.5: **G01N 33/574, G01N 33/68**

(30) Priority: **10.01.89 US 295550**

(43) Date of publication of application:
**18.07.90 Bulletin 90/29**

(84) Designated Contracting States:
**AT BE CH DE DK ES FR GB GR IT LI LU NL SE**

(71) Applicant: **ARIZONA BOARD OF REGENTS on behalf of THE UNIVERSITY OF ARIZONA Administration Building Room 103 Tucson Arizona 85721(US)**

(72) Inventor: **Dalton, William S.**
**6539 Camino Abbey**
**Tucson Arizona 85718(US)**
Inventor: **Grogan, Thomas M.**
**2025 East Fifth Street**
**Tucson Arizona 85718(US)**
Inventor: **Rybski, James A.**
**855 East River Road Nr. 115**
**Tucson Arizona 85718(US)**

(74) Representative: **Armitage, Ian Michael et al**
**MEWBURN ELLIS 2 Cursitor Street**
**London EC4A 1BQ(GB)**

(54) **Method and device for detection, validation and quantitation of multiple-drug resistance-associated antigens.**

(57) Method and device for the detection, validation and quantitation of antigens associated with multiple-drug resistance (MDR) for the purpose of predicting the degree of MDR of in vivo tumors, using MDR tumor cell lines as standards, affinitystaining, and a means of quantitating an MDR-associated antigen in individual cells.

Western Blot (PRIOR ART)

% Positive

Cytospin

Image Analysis

FIG.-1.

# METHOD AND DEVICE FOR DETECTION, VALIDATION AND QUANTITATION OF MULTIPLE-DRUG RESISTANCE-ASSOCIATED ANTIGENS

The present invention relates to the detection, validation and quantitation of cellular antigens; and more particularly to the detection, validation and quantitation of antigens associated with multiple-drug resistance for the purpose of predicting the degree of multiple-drug resistance of in vivo tumors.

There are a number of neoplasms with high initial response rates in vivo to chemotherapy where Vinca alkaloids, anthracyclines, or other cytotoxic drugs are employed. Over the course of treatment, however, these neoplasms become resistant to the particular drug(s) used. Interestingly, the development of resistance to the drug(s) used is accompanied by resistance to other drugs such that a second round of therapy with these other drugs is of little benefit. This type of resistance is described as multiple-drug resistance or MDR. It is thought that determining mechanisms of drug resistance and developing means of identifying drug resistant in vivo tumors could provide the basis for important advances in the treatment of neoplastic disease.

One of the first approaches to understanding MDR was the development and study of in vitro-established, MDR tumor cell lines. R.L. Juliano and V. Ling, Biochim. Biophys. Acta 455:152 (1976). R.J. Riordan and V. Ling, J. Biol. Chem. 254:12701 (1979). P.G. Debenham et al., Mol. Cell Biol. 2:881 (1982). N. Kartner et al., Science 221:1285 (1983). N. Kartner et al., Nature 316:820 (1985). W.S. Dalton et al., Cancer Res. 46:5125 (1986). These in vitro studies revealed a number of important findings. First, the MDR phenotype is associated with the over-expression of a tumor antigen, a 170,000 dalton membrane glycoprotein called P-glycoprotein or P-170. Second, the level of expression of this MDR-associated antigen is in a proportional relationship to the level of MDR.

From the handful of human in vivo studies to date it appears that these two in vitro findings extend to in vivo neoplasms. P-170 has been detected on MDR ovarian tumors. D.R. Bell et al., J. Clin. Onc. 3:311 (1985). P-170 has also been reported on MDR sarcomas. J.H. Gerlach et al., J. Clin. Onc. 5:1452 (1987). P-170 has, in addition, been described in the blast crisis of chronic myelocytic leukemia. G. Carulli et al., N. Eng. J. Med. 319:797 (1988). Finally, P-170 has been found on MDR non-lymphoblastic leukaemia in vivo. D.D.F. Ma et al., Lancet 1:135 (1987). In this latter case, the researchers showed that P-170 expression increased over the course of the disease.

It has been hoped that MDR-associated antigens such as P-170 would be of some aid to treatment strategy. A number of questions persist, however, as to how this can be done. First, there is the question of assay reliability or validation of method. To impact treatment strategy, physicians must be confident that false positives and false negatives are minimized. Second, both in vitro and in vivo studies reveal a spectrum of P-170 expression and a spectrum of drug resistance. The expression of P-170 can be quite heterogeneous within a tumor specimen, some cells staining very strongly while others staining weakly, or not at all. Thus, determining merely the presence or absence of P-170 detection, alone, does not allow for the quantitation of antigen expression and, moreover, is not sufficient to predict the degree of MDR of the particular in vivo tumor.

The present invention provides a method by which MDR-associated antigens such as P-170 aid in treatment strategy. The method is extremely reliable and allows for the prediction of the degree of MDR of patient tumors in vivo on the basis of MDR-associated antigen expression. By providing this validating and predictive tool, the invention represents an important advance in the treatment of MDR diseases.

Very significant treatment advantages are presented by this invention. By predicting the degree of MDR, this invention can enable a physician to choose cytotoxic drugs as therapy or cytotoxic drugs in combination with MDR-reversing drugs as therapy. A number of MDR-reversing drugs have been reported in in vitro studies. Tsuruo et al., Cancer Res. 41:1967 (1981). Tsuruo et al., Cancer Res. 43:2267 (1983). Tsuruo et al., Cancer Res. 43:2905 (1983). Rogan et al., Science 224:994 (1984). W.T. Beck, Advances Enzyme Regul. 22:207 (1984). Dalton et al., Proc. Am. Soc. Clin. Oncol. 6:31 (1987). B.G.M. Durie and W.S. Dalton, Brit. J. Haematol. 68:203 (1988). It is hoped that some of these drugs may have MDR-reversing properties when used in vivo.

Objects and advantages other than those above set forth will be apparent from the following description when read in connection with the accompanying figures.

The present invention is a method and device for predicting the degree of multiple drug resistance of tumor cells in vivo.

The method of the present invention comprises the steps:

a) affinitystaining a sample of tumor cells from an in vitro-established tumor cell line whose degree of multiple drug resistance is known:

b) affinitystaining a sample of the in vivo tumor cells;

c) quantitating the staining levels of said affinitystaining of steps a) and b); and

d) comparing said quantitated level of said affinitystaining of step a) with said quantitated level of said affinitystaining of step b), thereby predicting the degree of multiple drug resistance of said in vivo tumor cells.

In other embodiments, the method of the present invention comprises the steps:

a) delivering to the same analysis substrate:
i) at least one sample of tumor cells from an in vitro-established tumor cell line, and
ii) at least one sample of said in vivo tumor cells;

b) affinitystaining the samples on said analysis substrate;

c) quantitating the staining levels of said affinitystained samples on said analysis substrate; and

d) comparing said quantitated levels, thereby predicting the degree of multiple drug resistance of said in vivo tumor cells.

In still other embodiments, the method of the present invention comprises the steps:

a) affinitystaining:
i) at least one sample of tumor cells from an in vitro-established tumor cell line, and
ii) at least one sample of said in vivo tumor cells;

b) delivering said affinitystained samples to the same analysis substrate;

c) quantitating the staining levels of said affinitystained samples on said analysis substrate; and

d) comparing said quantitated levels, thereby predicting the degree of multiple drug resistance of said in vivo tumor cells.

In the embodiments where the samples are on the same analysis substrate, the delivering step comprises:

a) separately suspending each of said samples in a separate liquid medium;

b) separately containing said separately suspended samples;

c) providing a force to drive said separately contained and separately suspended samples in the direction of said analysis substrate; and

d) channeling said driven, separately contained and separately suspended samples to discrete and separate locations on said analysis substrate.

In some of the embodiments where the samples are on the same analysis substrate, the delivering step further comprises providing a device having:

a) means for separately suspending each of said samples in a separate liquid medium;

b) means for separately containing said separately suspended samples;

c) means for providing a force to drive said separately contained and separately suspended samples in the direction of said analysis substrate; and

d) means for channeling said driven, separately contained and separately suspended samples to discrete and separate locations on said analysis substrate.

The method of the present invention further provides a generalized method for delivering two or more distinct cell populations separately suspended in a liquid medium to the same analysis substrate. This generalized method comprises:

a) separately containing each of said separately suspended two or more distinct cell populations; and

b) channeling said separately contained each of said separately suspended two or more distinct cell populations to discrete and separate locations on said analysis substrate.

In some embodiments, the method for delivering cells to the same analysis substrate comprises using a delivering device. The delivering device is used to deliver two or more distinct cell populations separately suspended in a liquid medium to the same analysis substrate. The device comprises:

a) containing means for separately containing each of said separately suspended distinct cell populations; and

b) channeling means for channeling said separately contained, separately suspended distinct cell populations to discrete and separate locations on said analysis substrate.

By delivering the cells to the same analysis substrate, the method and device of the present invention allows for validation of the presence of MDR-associated antigen by accounting for false positive and negative results.

In the accompanying drawings:

Figure 1 shows the method of the present invention in a comparison with a commonly used prior art method (Western Blotting).

Figure 2 shows the resistance of MDR human tumor cells to doxorubicin.

Figure 3 shows the resistance of MDR human tumor cells to Velban.

Figure 4 shows the resistance of MDR human tumor cells to vincristine.

Figure 5 shows the resistance of MDR human tumor cells to VP-16.

Figure 6 shows the quantitation of MDR-associated antigen on in vitro established MDR human tumor cells.

Figure 7 shows the correlation between the degree of drug resistance and P-170 expression on in vitro established MDR human tumor cells.

Figure 8 shows a perspective view of one embodiment of a delivering device of the present invention.

Figure 9 is a photomicrographic comparison of MDR-associated antigen expression in an in vivo human tumor patient sample with MDR-associated antigen expression in in vitro established MDR cell lines.

Figure 10 shows a quantitative comparison of the P-170 expression of in vivo human tumor cells with in vitro established, MDR cell lines.

Figure 11 shows the resistance of in vivo MDR human tumor cells to doxorubicin, confirming the prediction of the degree of multiple drug resistance of tumor cells in vivo based on the MDR-associated antigen quantitation of the present invention.

The invention can be characterized as having five integral parts. First, the invention utilizes samples from in vivo tumors. Second, the invention utilizes affinitystaining to detect the MDR-associated antigen. Third, the invention involves the use of a quantitation system that assays individual cells within a population of tumor cells. Fourth, the invention takes advantage of in vitro-established, MDR tumor cell lines as a means of standardization and validation. Finally, the invention involves a quantitative comparison of affinitystained, in vivo tumor cells with affinitystained, in vitro-established, MDR tumor cell lines in order to, thereby, allow for the prediction of the degree of MDR of the in vivo tumor cells.

It should be noted that, while there are five parts to the present invention, a sixth part is necessary in order to confirm that the method predicts the degree of MDR. Confirmation is important, however, only to the extent that it serves to establish the accuracy of the prediction. It is not intended that confirmation be an integral part of the invention. Indeed, it is hoped that, through the established accuracy of the present invention, the labor and expense of conventional confirmation techniques can be avoided.

### 1. In Vivo Tumor Samples

The type of in vivo tumor sample and the specific method of collection depends on the particular type of in vivo tumor. For example, bone marrow aspirates are collected in heparin from myeloma patients. By contrast, lymph node biopsies are obtained from patients with lymphoma. On the other hand, either tumor nodules, involved lymph nodes or heparinized malignant effusions are obtained from breast cancer patients.

These three examples provide illustration of the methods used to assess myelomas, lymphomas and breast tumors. The present invention contemplates assessing any tumor type and is not limited to these examples. Many other tumors (ovarian, colon, etc.) may be examined. No matter what the histopathological classification of the tumor, all samples from human in vivo tumors are obtained during clinically indicated biopsy procedures and secondarily would be available for MDR quantitation.

It should also be noted that the present invention also contemplates the use of tumor cells that have been manipulated so as to isolate, eliminate or enrich a cellular or subcellular component. An example of such a manipulation contemplated by the invention is the preparation of membrane vesicles. M.M. Cornwell et al., Proc. Nat. Acad. Sci. 83:3847 (1986). The use of the words "cell" or "cells" is, therefore, meant to include such manipulated cells.

### 2. MDR-associated Antigen Detection

The invention is specifically designed to quantitate the level of P-170 detected on patient tumor samples so as to predict the degree of MDR of in vivo tumors. However, the invention is also applicable to quantitate the level of other MDR-associated antigens. P-170 is simply the best-characterized MDR-associated antigen thusfar. It appears that there is at least one family of genes that encode gene products that prove to be MDR-associated. Van der Bliek, et al., Mol. Cell. Biol. 6:1671 (1986). As the various mechanisms for drug resistance are elucidated, other gene products may be identified that are MDR-associated. In this regard, it should be noted that not all MDR cells express P-170. W.T. Beck et al., Cancer Res. 47:5455 (1987). Non-P-170-expressing MDR cells may express distinctly different MDR-associated antigens. The present invention contemplates the detection and quantitation of these distinct MDR-associated antigens as well.

A number of methods can be used to detect an MDR-associated antigen such as P-170 on tumor cells. Many of these methods, however, are ill-suited to the quantitation of P-170 for the purpose of predicting the degree of MDR. Extraction techniques that measure total P-170 mRNA from a population of cells, for example, are inappropriate for this purpose. A.T. Fojo et al., Proc. Nat. Acad. Sci. 84:265 (1987). First, the mRNA may never be translated to functional protein and therefore may have no relationship to MDR. Indeed, this may be the case with normal tissue, where P-170 mRNA has been described. Second, the total mRNA of a population of cells may show an overall low level of expression for the population and fail to show a high level of expression in a discreet subpopulation.

This latter problem is an important one and is due to the heterogeneity of cells in a tumor population. This problem is shared by other extraction methods such as the extraction of total P-170 protein from a population of cells followed by PAGE or followed by PAGE and Western Blotting. N. Kartner et al., Science 221:1285 (1983).

The present invention utilizes a particular method of MDR-associated antigen detection that accommodates the heterogeneity of cells in a tumor population. Detection of MDR-associated antigens such as P-170 is achieved by affinitystaining.

Affinitystaining comprises contacting cells with molecules that have affinity for MDR-associated antigens. These molecules, having the capability to directly bind with MDR-associated antigens, are hereinafter referred to as "direct-binding-molecules."

Direct-binding-molecules may be antibodies. However, direct-binding-molecules are not limited to antibodies and may be any type of molecule with the appropriate affinity and specificity. For example, lectins have been shown to bind to MDR-associated antigens. T. McGrath and M.S. Center, Cancer Res. 48:3959 (1988). Furthermore, drugs and drug-analogs have been demonstrated to bind to MDR-associated antigens. M.M. Cornwell et al., Proc. Nat. Acad. Sci. 83:3847 (1986). The present invention contemplates the use of lectins and drugs, as well as other type of compounds, for direct-binding-molecules.

The direct-binding-molecules used in affinitystaining may be labelled or unlabelled. If labelled, the direct-binding-molecules will have at least one label attached or integrated into its structure by conventional methods. Attachment may be by covalent or noncovalent bonds. Integration may be achieved during the synthesis of the direct-binding-molecule.

If the direct-binding-molecules are unlabelled, affinitystaining further comprises the addition of molecules with affinity and specificity for the direct-binding-molecules, hereinafter "indirect-binding-molecules." Indirect-binding-molecules may also be antibodies. However, indirect-binding-molecules are not limited to antibodies. For example, in the case where the direct-binding-molecules are antibodies, the indirect-binding-molecules may be any molecule with affinity and specificity for antibodies (such as Protein A, complement, cell receptors, serum factors, etc.).

It should be pointed out that use of techniques and compounds to enhance 1) binding of direct-binding-molecules to the cells, 2) binding of indirect-binding-molecules to direct-binding-molecules, and/or 3) attachment of labels to either direct-binding-molecules or indirect-binding-molecules, are also contemplated by the present invention. In the case of the attachment of labels, for instance, it is known that biotin-conjugated molecules can have labels attached by means of avidin-conjugated labels.

Indirect-binding-molecules may also be labelled or unlabelled. If labelled, the direct-binding-molecules will have at least one label covalently or noncovalently attached or integrated into its structure. If unlabelled, affinitystaining may further comprise additional molecules with affinity and specificity for either the direct-binding-molecules or the indirect-binding-molecules. The invention contemplates no limits, other than practical limits, on this stacking of binding molecules on one another.

Labels are intended to enhance the detection of direct-binding-molecules contacting cells by generating a signal. Labels, whether on direct-binding-molecules or on indirect-binding-molecules, are chosen from the group consisting of radioactive atoms, enzymes, fluorophores and chemiphores. Radiolabels commonly used include $^3$H, $^{14}$C, and $^{125}$I. Enzymes commonly used include horseradish peroxidase, alkaline phophatase, B-galactosidase and glucose oxidase. Fluorescein is the most commonly used fluorphore. The most commonly used chemiphore is the chemiluminescent dye, luminol.

Various methods or protocols may be employed in measuring the amount of labels (e.g. autoradiography, ELISA, etc.). Depending on which label is employed, further manipulations of the label, including additions of compounds, may be necessary to generate a detection signal. For example, where the label is an enzyme, it may be necessary to add substrate to generate a signal.

To detect P-170 on individual cells, the preferred approach is to use a monoclonal antibody, with affinity and specificity for a highly conserved epitope on P-170, as the direct-binding-molecule. In one embodiment of the invention, tumor cells are reacted with this antibody. Unbound antibody is washed away and bound antibody is detected by standard and well-known labelling systems.

EXAMPLE 1

To detect P-glycoprotein on individual cells, the murine monoclonal antibody JSB-1 (subclass IgG$_1$) is used as the direct-binding-molecule. R. Scheper et al., Int. J. Cancer 42:389 (1988). The biotin-avidin conjugated immunoperoxidase method of Warnke et al. is employed with some modifications. R. Warnke et al., Am. J. Clin. Pathol. 70:867 (1978). Tumor cells are suspended in RPMI media at approximately 200,000 cells/ml and 4 drops are placed in a cytocentrifuge apparatus (Cytospin 2, Shandon C., Sewickley, PA) along with one drop of 22% bovine serum albumin (Gamma Biological, Houston, TX). Cytocentrifuge preparations are dipped in cold acetone (4°C) for less than 5 seconds, air-dried and stored at -70°C. JSB-1 antibody from ascitic fluid is used at a dilution of 1/200 in 2% BSA in phosphate buffered saline. For the indirect-binding-molecule, biotin-conjugated rabbit anti-mouse IgG (H&L) (Accurate Chemical, Westbury, NY) is diluted to 1/1200 in 2% normal human serum in phosphate buffered saline. An enzyme label is subsequently attached using avidin-D conjugated with horseradish peroxidase (Vector Labs, Burlingame, CA). The color reaction is produced using a substrate, diaminobenzidine (Sigma Co., St. Louis, MO). After 5 minutes, 0.5% copper sulfate in 0.85% NaCl is used to intensify the color. A negative control slide is employed where all the above steps are repeated except that an irrelevant, isotype matched, monoclonal antibody is used instead of the specific monoclonal antibody.

EXAMPLE 2

All steps are performed as in Example 1 except that monoclonal antibody C-219 is used instead of antibody JSB-1. N. Kartner et al., Nature 316:820 (1980).

EXAMPLE 3

All steps are performed as in Example 1 except that monoclonal antibody MRK16 is used instead of antibody JSB-1. M.C. Willingham et al., J. Histochem. Cytochem. 35:1451 (1987).

EXAMPLE 4

All steps are performed as in Examples 1, 2 or 3 except that a nuclear counterstain is used as a final step.

3. Quantitation System

The level of affinitystaining intensity for an MDR-associated antigen in individual cells is quantitated visually and by measuring the energy absorption, reflection, refraction, or transmission characteristics of each cell. By measuring the affinitystaining levels of individual cells the problem of population heterogeneity is avoided. Furthermore, by using a technique which measures MDR-associated antigen in individual cells, one can also perform a differential on tumor specimens to determine not only the relative affinitystaining intensity of individual cells, but the percent of tumor cells which are positive. In this regard, small subpopulations of cells or even single cells expressing the MDR-associated antigen can be detected and quantitated.

The latter point is critical from a treatment standpoint. It is thought that drug resistance may arise from the selection pressure of cytotoxic drugs, i.e. those cells that are sensitive to the drugs die, while those cells that are resistant to the drugs replicate and gradually become the predominant cell population. Measurement of small, MDR-associated antigen-positive subpopulations would reveal stages in this process. Treatment choices will vary according to the stage of drug resistance identified.

EXAMPLE 5

Cells are affinitystained with an antibody specific for P-170 as described in Examples 1, 2 or 3 and then examined with the CAS 100 optical microscope image analysis system. J.W. Bacus and L.J. Grace, Applied Optics 26:3280 (1987). The CAS 100 system consists of a Reichert-Jung Diastar model 420 microscope (Cambridge Instruments) with the microscopic image collected by a charge-coupled device (CCD) video camera. The CCD is attached to the microscope and cellular images are sent to an image processing board contained within an 8 megaHertz Intel 80286 central processing unit based microcomputer containing 512 kilobytes of random access memory, a math co-processor and an internal 30 megabyte fixed-disk. Digitized images are displayed directly on a Barco high-resolution RGB monitor directly under software control. The CAS 100 and its software can be purchased from Becton-Dickinson Immunocytometry Systems.

Each cell's optical density (which represents the sum optical density/total number of pixels in each cell's image) is determined using the 40x objective and a 480nm filter (20nm bandwidth) and the cell measurement program (CMP) purchased from Becton-Dickinson. In some experiments, the data are expressed as the mean optical density ± SEM for approximately 50 cells per sample.

EXAMPLE 6

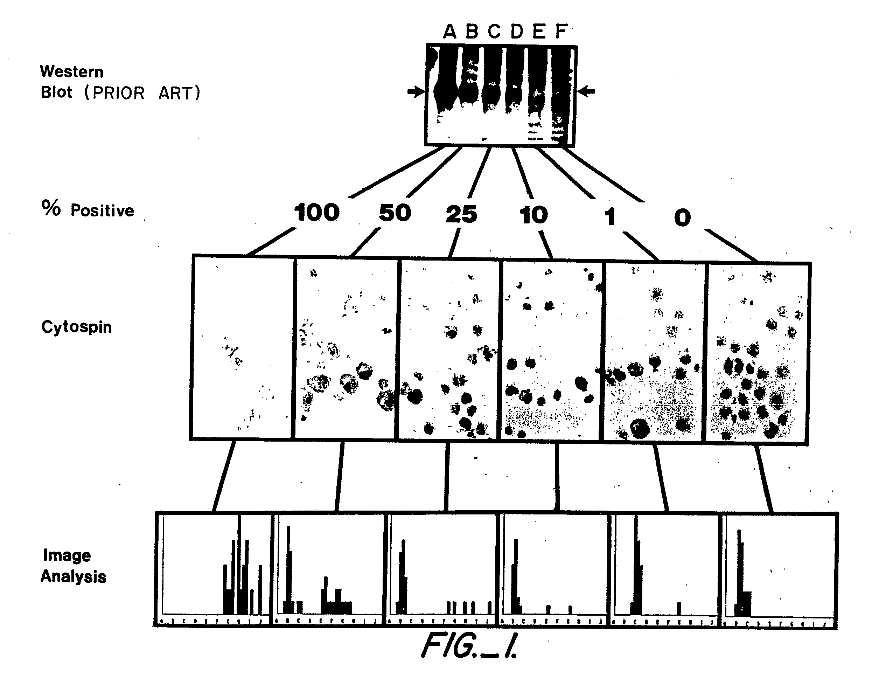

A cell dilution study is performed to compare the sensitivity of the quantitation method of the present invention with prior art quantitation methods. Figure 1 shows the affinitystaining and quantitation methods of the present invention compared to a commonly used prior art method. Six myeloma cell populations, each having a different concentration of P-170 positive cells (100, 50, 25, 10, 1 and 0%), are prepared by either 1) affinitystaining and quantitation according to the present invention (Examples 1 and 5, respectively) or 2) extraction of total P-170 protein followed by PAGE and Western Blotting. N. Kartner et al., Science 221:1285 (1983). (The total cell number is constant for all the preparations).

The results are depicted in three ways: 1) the prior art method (Western Blot) is shown above 2) six photomicrographs of cells that have been cytocentri fuged (Cytospin) and affinitystained (Example 1), which are shown above 3) six x/y plots of the image Analyzer (Example 5). Each lane of the six lanes of the Western Blot corresponds with each photomicrograph of the six photomicrographs, which corresponds with each x/y Image Analysis plot, representing the same cell population.

The far left-hand lane (Lane A) of the Western Blot shows a heavy band (arrow) at approximately 170,000 M.W. (gel molecular weight markers are not shown) that is characteristic of P-170. Lanes B through D show the same band, although the band appears in decreasing intensity. Lanes E and F show no band (arrow). At most, the Western Blot suggests that each population has less P-170 protein expressed. From the gel there is no way to tell a) the specific percentage of cells expressing P-170, b) whether expression is uniform, and, most importantly, c) that the population of Lane E has a small population of cells (1%) expressing P-170.

The corresponding photomicrographs, by contrast, provide all this information. Microscopic examination of affinitystained cells according to the present invention (Example 1) allows for a calculation of the specific percentage of cells expressing P-170 within a field of vision. Furthermore, the relative expression of P-170 can be determined on an individual cell basis. Most importantly, small subpopulations of cells or even single cells expressing the MDR-associated antigen can be detected and quantitated; P-170 is detected even when only 1% of the population expresses the protein. (Note: the affinitystaining of Figure 1 is seen most clearly in the color photomicrographs submitted with the patent application. Black and white reproduction in the patent printing process may cause the photomicrograph to lose its contrast features.)

The quantitation system of the present invention allows for greater precision in determining the relative expression and percent expression of P-170. The x axis of the Image Analysis shows staining intensity. The y axis shows cell number. An example of discriminating power of the system is shown in the plot corresponding to 50% expression which shows that there are two distinct subpopulations within the population: 1) a subpopulation with a high level of staining, and 2) a subpopulation with a low to medium level of staining.

In Examples 5 and 6, it should be noted, the level of affinitystaining intensity for an MDR-associated antigen in individual cells is quantitated visually and by measuring the average optical density for each cell. In these examples, therefore, the energy characteristic measured is the degree of opacity of each cell, which is commonly called optical density and is expressed by log $I_o/I$, where $I_o$ is the intensity of the incident ray, and $I$ is the intensity of the transmitted ray. As noted above, the present invention contemplates quantitating the level of affinitystaining intensity by measurement of any energy absorption, reflection, refraction, or transmission characteristic of each cell. Thus, the present invention is not intended to be limited to the particular measurement technique used in the Examples.

Because the present invention contemplates quantitating the level of affinitystaining intensity by measurement of any energy absorption, reflection, refraction, or transmission characteristic of each cell, the present invention necessarily contemplates placing the cells on an analysis substrate that is appropriate for such measurements. By this it is meant that the analysis substrate does not impact the energy contacting it so as to make measurement of the energy impossible. For example, where the energy is light, the analysis substrate might be transparent. By this it is also meant that the analysis substrate supports the cells without interacting with the cells so as to make measurement of the cells impossible. Examples of such non-interacting analysis substrates are those made from glass or plastic.

With these desired properties in mind, a number of analysis substrates can be contemplated for use. Examples include (but are not limited to) petri dishes, multiwell plates, and microscope slides. Normally, the analysis substrate will be unmodified. However, the invention also contemplates use of a modified analysis substrate such as protein-coated or polymer-coated, glass or plastic. The various reasons for the use of a modified analysis substrate include (but are not limited to) combating the natural surface charge of the analysis substrate, stabilizing the environment for the cells, and/or anchoring the cells to the analysis substrate.

## 4. Standardization with MDR Cell Lines

The invention involves a method to quantitate the amount of MDR-associated antigens on a sample of individual cells from a population of in vivo tumor cells after affinitystaining, and comparing the values obtained to affinitystained, in vitro-established tumor cell lines expressing known amounts of MDR-associated antigen (positive standards) and those without MDR-associated antigen (negative standards).

The invention contemplates using 1) in vitro-established tumor cell lines that are of the same histopathological type as the in vivo tumor cells, 2) in vitro-established tumor cell lines that are not of the same histopathological type as the in vivo tumor cells, or 3) both histopathologically similar and histopathologically dissimilar in vitro-established tumor cell lines. Most importantly, the in vitro-established tumor cell lines are characterized with respect to the level of drug resistance.

## EXAMPLE 7

RPMI 8226/S myeloma cells obtained from the American Type Culture Collection (Rockville, MD), are selected for resistance to doxorubicin (Dox) (Adria Labs, Columbus, OH) by gradually increasing Dox exposure over a 2 year period. W.S. Dalton et al., Cancer Res. 46:5125 (1986). The 8226/$\overline{Dox_6}$ cell line is selected for resistance by gradually exposing the cells to $6 \times 10^{-8}$M Dox, a concentration of six times the initial concentration ($1 \times 10^{-8}$M) used in the selection process. The 8226/$Dox_{40}$ cells are gradually exposed to a maximum concentration of $4 \times 10^{-7}$M Dox, representing a 40-fold increase in the initial drug exposure concentration. Similarly, the 8226/$Dox_{60}$ cells are gradually exposed to a maximum concentration of $6 \times 10^{-7}$ M Dox, representing a 60-fold increase in the initial drug exposure concentration.

Cells are maintained as a suspension in RPMI 1640 medium supplemented with 10% (v/v) fetal bovine serum, 1% penicillin (100 $\mu$/ml), 1% streptomycin (100$\mu$g/ml) and 1% (v/v) L-glutamine (Grand Island Biological Co., Grand Island, NY). Cells are kept at 37°C in a 5% $CO_2$-95% air atmosphere and subcultured every 7 days. Once established, resistance is stable in the doxorubicin-resistant cell lines for at least one year.

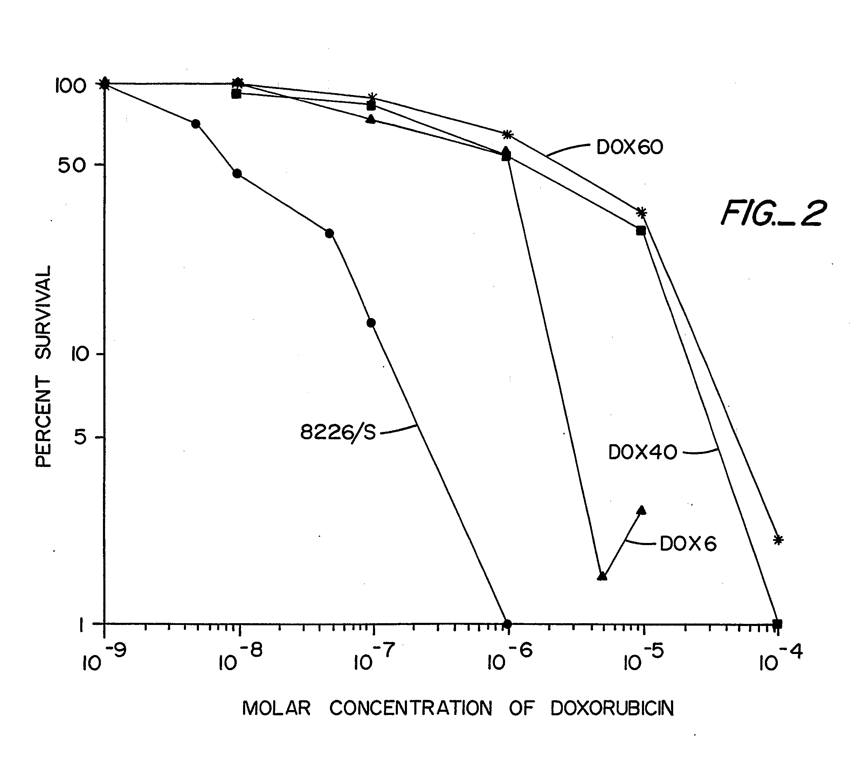

The cell survival curves of the five 8226 cell lines are determined after continuous exposure to varying doses of doxorubicin in soft agar following a modification of the plating technique of A.W. Hamburger and S.E. Salmon, Science 197: 461 (1977) (Figure 2). Exponentially growing cells were exposed to varying concentrations of cytotoxic, anticancer drugs for 1 h at 37°C. At the end of 1 h the cells were washed and plated in triplicate in 35-mm petri dishes at 20,000 cells per plate. Plates were then incubated at 20,000 cells per plate in a humidified atmosphere of 5% $CO_2$ at 37°C for 10 days. Colonies greater than 6u $\mu$m were counted by inverted microscopy or by computerized image analyzer (Omincon FAS II; Bausch and Lomb, Inc. Rochester, NY). The concentration of drug which produced a 50% inhibition of cloning efficiency ($IC_{36}$) was calculated from linear transformation of the dose response curves. The mean lethal dose ($D_o$) doxorubicin required to reduce survival to 1/e (37%) of the initial cell population was determined using the negative reciprocal slope of the linear portion of the survival curves. The values (not shown) reflect, in the case of the 8226/$Dox_6$ and 8226/$Dox_{40}$ cell lines for example, a 10-fold and 61-fold increase in resistance, respectively.

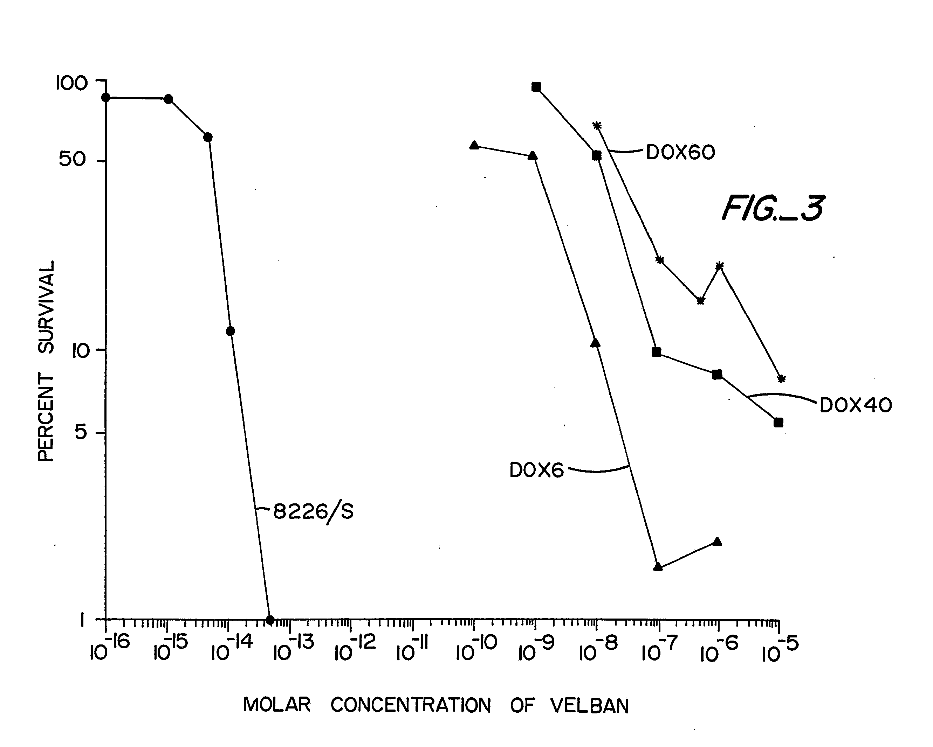

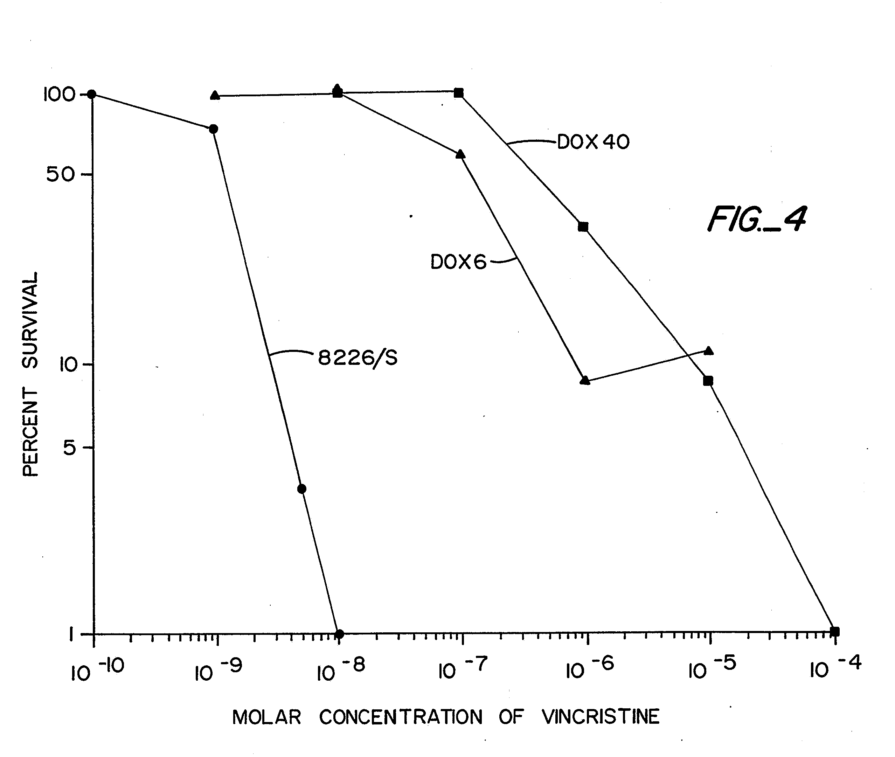

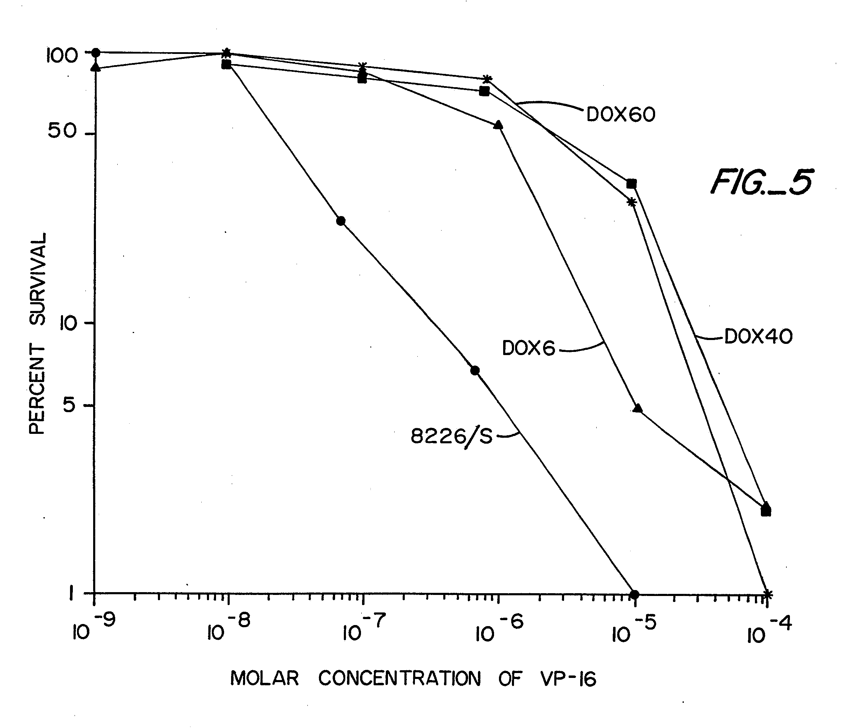

The MDR profile for each of the resistant myeloma cell lines is characteristic of multidrug-resistant cell lines, exhibiting resistance to many drugs in addition to doxorubicin, such as Velban (Figure 3), vincristine (Figure 4) and VP-16 (Figure 5), but increased sensitivity to steroids, including dexamethasone (not shown).

Using the computerized cell analysis system (CAS) described above, the amount of staining intensity for the individual cells was quantified by measuring the average optical density of each cell. In Figure 6, the mean optical density was measured for approximately 50 cells from each of the drug-resistant cell lines (8226/$Dox_6$, 8226/$Dox_{40}$ and 8226/$Dox_{60}$) after affinitystaining with monoclonal antibody C-219 (Example 2, above). The intensity of staining for individual cells within each cell line was uniform (data not shown). Importantly, from Figure 6 it appears that the drug dose used to cause resistance correlates with the expression of P-170.

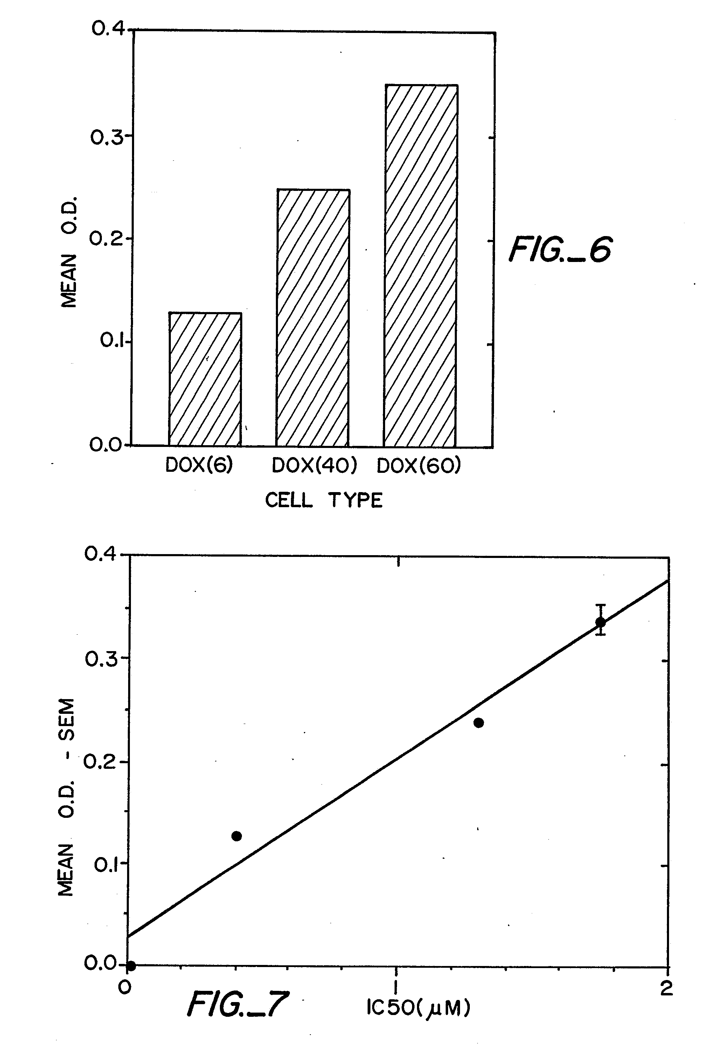

In Figure 7, linear regression analysis was performed on individual cell optical density values and related to the mean lethal dose, D0, of individual cell lines (8226/S, 8226/$Dox_6$, 8226/$Dox_{40}$ and 8226/$Dox_{60}$) after affinitystaining with monoclonal antibody C-219 (Example 2, above). Linear regression analysis of all data points revealed a strong correlation (r = 0.901) between the level of P-glycoprotein (optical density) and mean lethal dose ($D_o$) for each individual cell line. Thus, the level of drug resistance in these cell lines is strongly correlated with the level of expression of P-170.

It should be noted that, for the best correlations using this particular measuring technique, the cells should not be counterstained (see Example 4). It is observed that counterstaining interferes with the CAS 100's ability to measure cellular optical density by lowering the signal-to-noise ratio. With these considerations, the antibody threshold can be set such that the optical density of 8226/$_s$ (i.e., the drug-sensitive cells) equals zero.

EXAMPLE 8

Mitoxantrone-resistant, human colon carcinoma cell lines are established by continuously exposing cells to gradually increasing concentrations of mitoxantrone. R.E. Wallace et al., Proc. Am. Assoc. Cancer Res. 23:767 (1982). Cultures are grown as a monolayer in Eagle's minimum essential medium (Grand Island Biological Co.) supplemented by 10% (v/v) fetal bovine serum (Hyclone Co.), 2.0% (v/v) 20mM L-glutamine (Grand Island Biological Co.) and 1.0% (v/v) penicillin/streptomycin (10,000 units/ml; Grand Island Biological Co.). Cell lines are cultured at 37° C in 95% humidity, 5% $CO_2$ and 20% $O_2$.

The drug resistant profile for this resistant colon cancer cell line is characteristic of multidrug-resistant cell lines (data not shown). However, P-170 is not overexpressed. This demonstrates that the MDR phenotype may be associated with molecules which are antigenically distinct from P-170. W.S. Dalton et al., Cancer Res. 48:1882 (1988). The present invention contemplates predicting the degree of MDR on the basis of quantitating and correlating the expression of these distinct antigens.

EXAMPLE 9

Mitoxantrone-resistant, human breast carcinoma cell lines are established by continuously exposing cells to gradually increasing concentrations of mitoxantrone. Cultures are prepared as in Example 8.

The drug resistant profile for this resistant breast cancer cell line is characteristic of MDR cell lines (data not shown). But once again, P-170 is not overexpressed, indicating that different molecules may define the MDR phenotype. The present invention contemplates predicting the degree of MDR on the basis of quantitating and correlating the expression of these distinct antigens.

The best method for the above-described correlations involves the affinitystaining of all cells at the same time. This is best achieved by first delivering all the cells to be affinitystained to the same analysis substrate, such as a microscope slide. Affinitystaining all the cells on the same slide eliminates any run-to-run variability in staining intensity.

The particular number of cell populations to be affinitystained can vary. The present invention contemplates delivering to the same analysis substrate i) at least one sample of tumor cells from an in vitro-established tumor cell line, and ii) at least one sample of in vivo tumor cells.

While it is possible that the distinct cell populations delivered to the same analysis substrate are mixed, it is contemplated that the best method is to deliver the cell populations unmixed. To this end, it is contemplated that each cell population would be separately suspended in liquid medium. Again, the number of distinct cell populations suspended will vary. The present invention contemplates, however, separately suspending in liquid medium i) at least one sample of tumor cells from an in vitro-established tumor cell line, and ii) at least one sample of said in vivo tumor cells.

To maintain the unmixed state of the various cell populations the present invention contemplates separately containing the separately suspended cell populations. Again, the number of separately contained cell populations will vary. Nonetheless, the present invention contemplates separately containing i) at least one sample of tumor cells from an in vitro-established tumor cell line, and ii) at least one sample of said in vivo tumor cells.

Because the cell populations are initially suspended in a liquid medium, the present invention contemplates providing a force to drive the suspended cells to the contact surface of the analysis substrate. The force might be any force capable of driving the cells in this manner. For example, the force might simply be the natural force of gravity. A more convenient source of a force for this procedure is centrifugal force. Cytocentrifuge apparatus, such as the Cytospin 2 (Shandon Southern Instruments, Inc., Sewickley, PA) are commercially available. In any event, the present invention contemplates simultaneously driving the separately contained and separately suspended cell samples in the direction of the analysis substrate.

To maintain the unmixed state of the cell populations after a force is applied, the present invention contemplates simultaneously channeling the driven, separately contained and separately suspended cell samples to discrete and separate locations on the analysis substrate.

At the point where the cells have been driven to the analysis substrate, the liquid medium for each cell population will be largely devoid of cells. Even if the liquid medium is to be used in the affinitystaining process (for example, the in vivo and in vitro samples might be affinitystained in the liquid medium and then delivered to the same analysis substrate), the convenient approach is to remove the liquid medium. One method of removing the liquid medium might be to draw off the liquid medium by use of a suction or vacuum pump. A more convenient approach, however, involves absorbing the liquid medium.

EXAMPLE 10

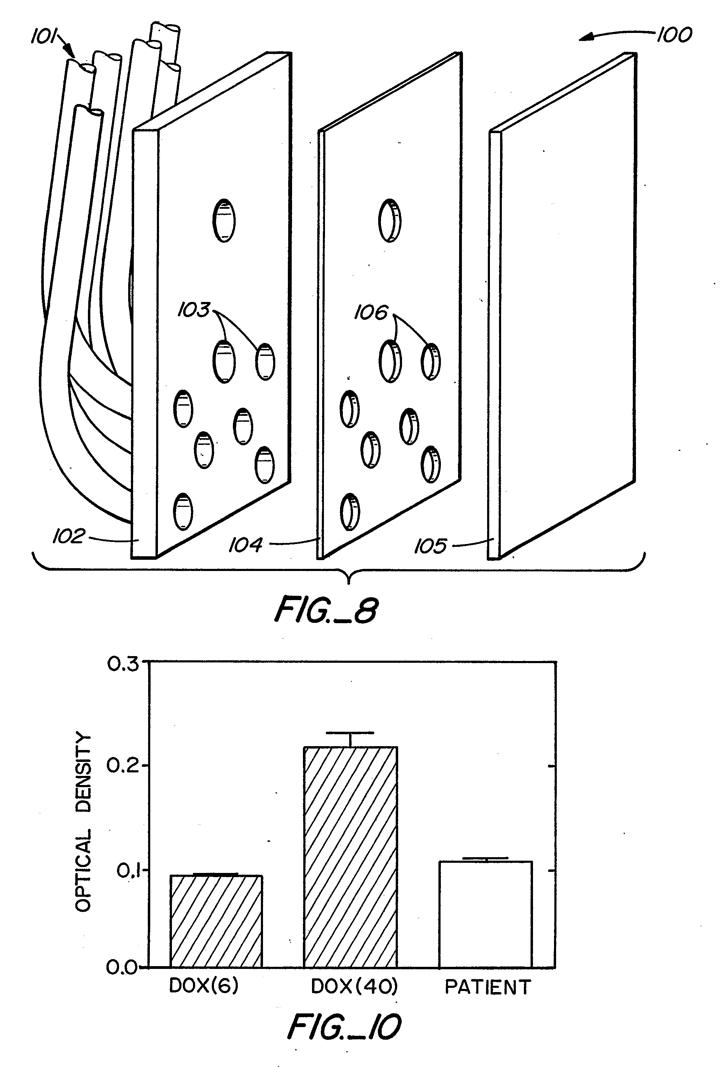

Delivering all the cells to be affinitystained to the same analysis substrate can also be achieved by using a delivering device. Figure 8 shows a perspective view of one embodiment of a delivering device (100) of the present invention. The device (100) comprises two or more tubes (101) connected to a base plate (102) having two or more perforations (103) adapted to fit the two or more tubes (101). The tubes (101) serve as a means for separately containing each of the separately suspended, distinct cell populations to be affinitystained (not shown). The perforations (103) serve as the means for channeling each of the separately contained and separately suspended two or more distinct cell populations (not shown) to discrete and separate locations on the analysis substrate (105).

Where it is desired that the device (100) removes the liquid medium (not shown), the device further comprises a filter pad (104). It is contemplated that the filter pad (104) is made out of material with absorbing properties.

The filter pad (104) might be permanently attached to the base plate (102) or might be detachable as shown in Figure 8. Importantly, whether permanently attached or detachable, the filter pad (104) is placed between the base plate (102) and the analysis substrate (105) and has two or more perforations (106) aligned with the two or more perforations (103) of the base plate (102).

5. Comparison and Prediction

The invention combines the above-described parts of the invention to compare the MDR-associated antigen expression of in vivo tumor samples with the MDR-associated antigen of in vitro-established, MDR tumor cell lines. Because of the known degree of MDR in the various MDR tumor cell lines and the correlation of this degree of MDR with the degree of MDR-associated antigen expression, the quantitation of in vivo tumor expression of MDR-associated antigen can predict the degree of MDR in the patient tumor.

EXAMPLE 11

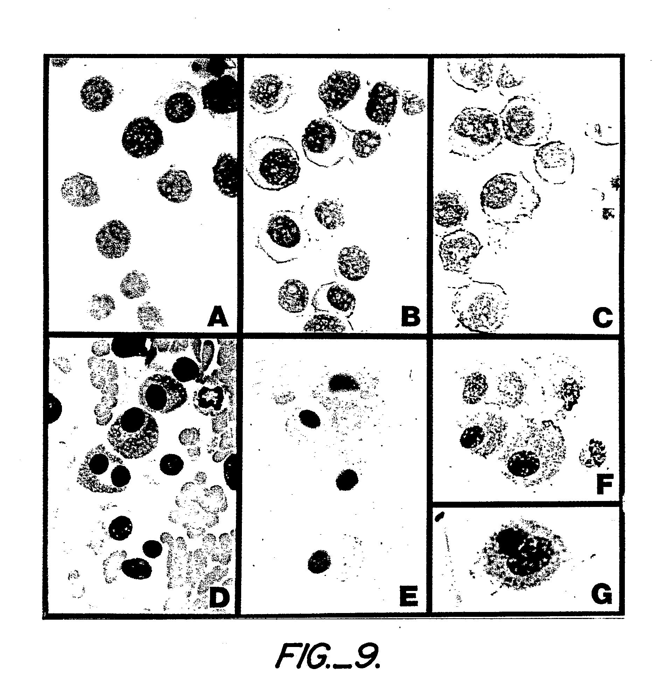

Figure 9 is a photomicrographic comparison of P-170 expression in a myeloma patient sample with P-170 expression in MDR cell lines using the monoclonal antibody JSB-1 as the direct-binding-molecule. The assay is performed generally as described in Example 1, above, including the use of negative controls. The MDR in vitro-established tumor cell lines serve as positive standards and are used along with a patient specimen. To compare the amount of P-glycoprotein on patient specimens with the amount on the cell lines, cells from both the patient and the MDR tumor cell lines are affinitystained simultaneously to control for variability in affinitystaining technique.

Figure 9A is a negative control for 8226/Dox$_{40}$. Figure 9B and 9C show the affinitystaining results for the drug resistant cell lines 8226/Dox$_6$ and Dox$_{40}$, respectively. As the cells become more resistant, the intensity of staining (brown coloration) of individual cells also increases. The staining is particularly intense in the plasma membrane of individual cells with occasional cells showing increased staining of the plasma cell "hof" (Golgi apparatus) (see Figures 9C and G). (Note: the affinitystaining of Figure 9 is seen most clearly in the color photomicrographs submitted with the patent application. Black and white reproduction in the patent printing process may cause the photomicrograph to lose its contrast features.)

The results of cell staining for a patient with drug refractory, end-stage multiple myeloma are shown in Figures 9D, E, F and G. Figure 9D shows the typical cell morphology of a patient with myeloma. The negative control for this patient's cells are seen in Figure 9E. Figures 9F and G are typical P-glycoprotein positive cells seen in this patient specimen.

The myeloma cells of 12 other patients with drug refractory disease have been examined in the same manner (not shown). Six of these patients had P-glycoprotein positive cells. All 12 patients tested had prior chemotherapy containing vincristine and doxorubicin and were considered to be drug refractory. Five of these patients were treated with combination chemotherapy consisting of continuous infusion vincristine and doxorubicin with oral decadron (VAD) in combination with verapamil.

EXAMPLE 12

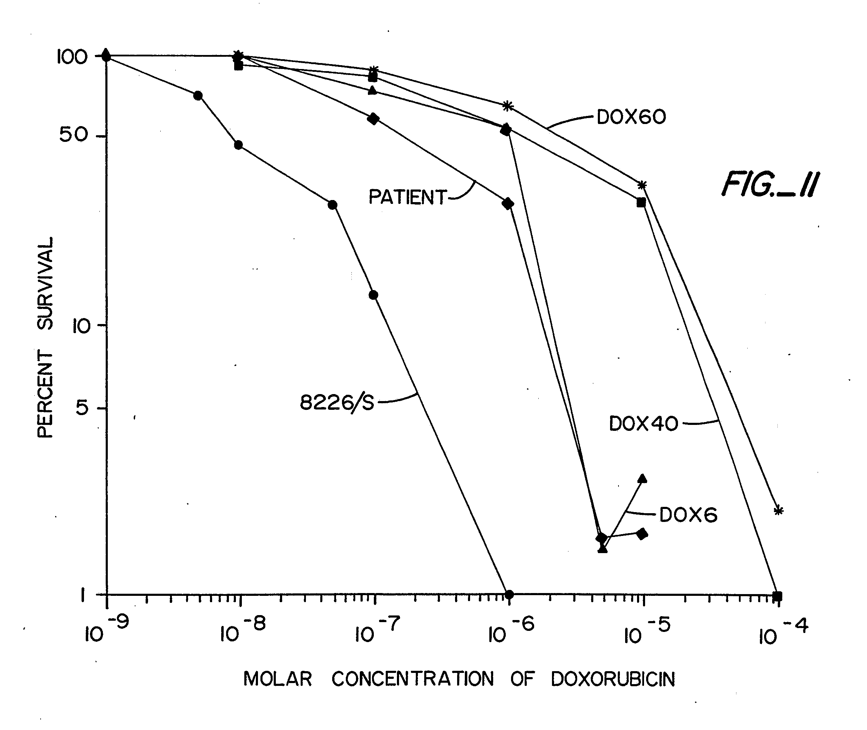

In Figure 10, the level of P-170 expression seen in a patient's specimen (Figure 9) is quantitatively compared to the level of P-170 expression observed in the in vitro MDR cell lines. This particular patient's cells appeared to have slightly more P-170 than the Dox₆ cell line. As noted above, the Dox₆ cell line is 10-fold resistant compared to 8226/S. Based on this quantitative comparison, it is predicted that the patient tumor cells have approximately the same degree of MDR as that observed for the Dox₆ cell line.

The best method of assuring the accuracy of the prediction involves affinitystaining of fresh in vivo tumor cell samples. In this regard, it is observed that the level of MDR-associated antigen expressed by patient in vivo tumor cells transferred to in vitro culture may vary over time. In contrast, the in vitro-established MDR cell lines are very stable in tissue culture in terms of MDR-associated antigen expression. Obviously, the extent to which the properties of the transferred patient cells become distinct from the properties of their in vivo counterpart will determine the extent to which reliance can be placed on measurements of the transferred cells.

### 6. Confirmation

As noted above, it may be desirable to initially confirm that the method accurately predicts the degree of MDR. Confirmation is important, however, only to the extent that it serves to establish the accuracy of the prediction. It is not intended that confirmation be an integral part of the invention. Indeed, it is hoped that, through the established accuracy of the present invention, the labor and expense of conventional confirmation techniques can be avoided.

### EXAMPLE 13

At the time of biopsy, the patient measured in Figure 10 was a 43 year old female patient with IgA, kappa multiple myeloma had developed progressive disease after treatment with multiple drugs including vincristine and doxorubicin. To confirm the prediction of Figure 10, the cell survival curves of the in vivo tumor cells and the in vitro-established MDR cell lines can be determined after continuous exposure to varying doses of doxorubicin in soft agar following a modification of the plating technique of A.W. Hamburger and S.E. Salmon, Science 197: 461 (1977). In this assay, exponentially growing cells are exposed to varying concentrations of the cytotoxic, anticancer drug for one hour at 37° C. At the end of one hour, the cells are washed and plated in triplicate in 35-mm petri dishes at 20,000 cells per plate. Plates are then incubated at 20,000 cells per plate in a humidified atmosphere of 5% $CO_2$ at 37° C for 10 days. Colonies greater than 60 $\mu$m are counted by inverted microscopy or by computerized image analyzer (Omincon FAS II; Bausch and Lomb, Inc. Rochester, NY). The data may be expressed as percent survival.

Referring to Figure 11, the survival curve for the in vivo tumor cells of the patient of Figure 10 falls on top of the survival curve of the Dox₆ cell line, thus confirming the prediction of the degree of multiple drug resistance of tumor cells in vivo based on the MDR-associated antigen quantitation of the present invention.

### EXAMPLE 14

Drug sensitivity testing may also be performed on the fresh, in vivo tumor cell suspensions and the in vitro-established tumor cell lines using a human tumor clonogenic assay using standardized procedures for tumor cell plating in semisolid medium (agarose) in the presence of complete medium containing 10% fetal calf serum. Doxorubicin sensitivity is assessed by adding the drug over a several log range (.01-1.0 $\mu$g/ml) which brackets pharmacologically achievable plasma doxorubicin concentrations. Triplicate 0.5 ml cultures containing 100,000 cells are prepared for control and drug containing cultures at each dosage level and incubated for 96 hours. Using a modification of the procedure reported by N. Tanigawa et al., Cancer Res. 42:2159 (1982), tritiated thymidine (at a final concentration of 10 $\mu$Ci/ml) is added to each culture for an additional 48 hours at which time the cultures are harvested for liquid scintillation counting of tritiated thymidine incorporation into acid precipitable DNA. Use of the tritiated thymidine endpoint rather than visual colony counting (see Examples 7 and 13, above) permits an earlier determination of results and also significantly increases the proportion of cases evaluable for drug sensitivity testing results.

### Claims

A method for predicting multiple drug resistance of tumor cells in vivo, comprising the steps:

a) affinitystaining a sample of tumor cells from an in vitro-established tumor cell line whose degree of multiple drug resistance is known;

b) affinitystaining a sample of the in vivo tumor cells;

c) measuring the affinitystaining levels of individual cells of steps a) and b); and

d) comparing the values obtained for individual cells of step a) to the values obtained for individual cells of step b) to predict multiple drug resistance of said *in vivo* tumor cells.

2. The method of claim 1 wherein said method detects multiple drug resistance in the sample of the *in vivo* tumor and the degree of multiple drug resistance of said *in vitro*-established tumor cell line is 0%.

3. The method of claim 1 wherein said method predicts the degree of multiple drug resistance of the sample of the *in vivo* tumor and of at least two samples of tumor cells from said *in vitro* established tumor cell line, said *in vitro* established tumor cell line samples having different degrees of multiple drug resistance, are affinitystained in step a) and the affinitystaining levels of individual cells of steps a) and b) are quantitated.

4. The method of claim 1 wherein the level of affinitystaining of individual cells is quantitated visually.

5. A method as recited in claim 1 wherein said *in vitro*-established tumor cell line is selected for resistance to a cytotoxic drug by gradually increasing exposure to said cytotoxic drug over a period of time.

6. A method as recited in claim 5 wherein said cytotoxic drug is selected from the group consisting of doxorubicin and mitoxantrone.

7. A method a recited in claim 1 wherein said in vitro-established tumor cell lines are of the same histopathological type as said *in vivo* tumor cells.

8. A method as recited in claim 1 wherein said quantitating comprises determining the average optical density of individual tumor cells after said affinitystaining.

9. A method as recited in claim 1 wherein said affinitystaining comprises contacting cells with antibodies having an affinity for an MDR-associated antigen.

10. A method as recited in claim 9 wherein said MDR-associated antigen is P-170.

A B C D E F

**Western**
**Blot (PRIOR ART)**

**% Positive**

**100    50    25    10    1    0**

**Cytospin**

**Image**
**Analysis**

*FIG._I.*

EP 0 378 383 A2

FIG._2

EP 0 378 383 A2

FIG._3

EP 0 378 383 A2

FIG._4

EP 0 378 383 A2

FIG._5

EP 0 378 383 A2

EP 0 378 383 A2

FIG._6

FIG._7

*FIG._8*

*FIG._10*

FIG._9.

FIG._II

EP 0 378 383 A2